# EUROPÄISCHE PATENTSCHRIFT

(11) **EP 2 147 313 B1**
(45) Veröffentlichungstag und Bekanntmachung des Hinweises auf die Patenterteilung: **03.08.2011**
(21) Anmeldenummer: 08758390.2
(22) Anmeldetag: 06.05.2008
(51) Int. Cl.: G01N 33/543, G01N 33/68

(54) **VERFAHREN ZUM NACHWEIS SPEZIFISCHER ANTIKÖRPER DER IMMUNGLOBULINKLASSE G**
METHOD FOR THE DETECTION OF SPECIFIC IMMUNOGLOBULIN CLASS G ANTIBODIES
PROCÉDÉ DE DÉTECTION D'ANTICORPS SPÉCIFIQUES DE LA CLASSE DES IMMUNOGLOBULINES G

(30) Priorität: 08.05.2007 EP 07009240
(43) Veröffentlichungstag der Anmeldung: 27.01.2010
(73) Patentinhaber: F. Hoffmann-La Roche AG, 4070 Basel (CH); Roche Diagnostics GmbH, 68305 Mannheim (DE)
(72) Erfinder: BRONOLD, Martina, 82515 Wolfratshausen (DE); MARKERT-HAHN, Christine, 82377 Penzberg (DE); DONIE, Frederic, 82377 Penzberg (DE); BOLLHAGEN, Ralf, 82377 Penzberg (DE); UPMEIER, Barbara, 82393 Iffeldorf (DE)
(74) Vertreter: Weiss, Wolfgang
(86) Internationale Anmeldenummer: PCT/EP2008/003623
(87) Internationale Veröffentlichungsnummer: WO 2008/135274

(56) Entgegenhaltungen:
- WO-A-98/12539
- US-A1- 2003 003 460
- YIN X-B ET AL: "4-(Dimethylamino)butyric acid labeling for electrochemiluminescence detection of biological substances by increasing sensitivity with gold nanoparticle amplification" ANALYTICAL CHEMISTRY 20050601 AMERICAN CHEMICAL SOCIETY US, Bd. 77, Nr. 11, 1. Juni 2005 (2005-06-01), Seiten 3525-3530, XP002495080
- MERRILL ET AL: "A quantitative electrochemiluminescence assay for Clostridium perfringens alpha toxin" ANALYTICAL BIOCHEMISTRY, ACADEMIC PRESS INC. NEW YORK, Bd. 357, Nr. 2, 15. Oktober 2006 (2006-10-15), Seiten 181-187, XP005660641 ISSN: 0003-2697
- SCHAERTL S ET AL: "A NOVEL AND ROBUST HOMOGENEOUS FLUORESCENCE-BASED ASSAY USING NANOPARTICLES FOR PHARMACEUTICAL SCREENING AND DIAGNOSTICS" JOURNAL OF BIOMOLECULAR SCREENING, LARCHMONT, NY, US, Bd. 5, Nr. 4, 1. Januar 2000 (2000-01-01), Seiten 227-237, XP001006052 ISSN: 1087-0571
- BENECKY M J ET AL: "Detection of hepatitis B surface antigen in whole blood by coupled particle light scattering (Copalis)" CLINICAL CHEMISTRY, AMERICAN ASSOCIATION FOR CLINICAL CHEMISTRY, WASHINGTON, DC, Bd. 43, Nr. 9, 1. Januar 1997 (1997-01-01), Seiten 1764-1770, XP002326863 ISSN: 0009-9147
- BYSTRYAK S ET AL: "HOMOGENEOUS IMMUNOFLUORESCENCE ASSAY BASED ON DYE-SENSITIZED PHOTOBLEACHING" ANALYTICAL BIOCHEMISTRY, ACADEMIC PRESS INC. NEW YORK, Bd. 225, 1. Januar 1995 (1995-01-01), Seiten 127-134, XP000882217 ISSN: 0003-2697
- KLAASSEN R J ET AL: "Differentiation between neutrophil-bound antibodies and immune complexes.", March 1991 (1991-03), BRITISH JOURNAL OF HAEMATOLOGY MAR 1991 LNKD- PUBMED:2012766, VOL. 77, NR. 3, PAGE(S) 398 - 402 ISSN: 0007-1048
- BOAS G: "Surface plasmon resonance nanosensors help identify biomarkers for Alzheimer's disease: Technique could lead to clinical detection of the disease", BIOPHOTONICS INTERNATIONAL, LAURIN PUBL., PITTSFIELD, MA, US, vol. 12, no. 4, 1 April 2005 (2005-04-01), pages 60-61, XP008098932, ISSN: 1081-8693
- KLAASSEN R J ET AL: "Differentiation between neutrophil-bound antibodies and immune complexes." March 1991 (1991-03), BRITISH JOURNAL OF HAEMATOLOGY MAR 1991 LNKD- PUBMED:2012766, VOL. 77, NR. 3, PAGE(S) 398 - 402 ISSN: 0007-1048
- BOAS G: "Surface plasmon resonance nanosensors help identify biomarkers for Alzheimer's disease: Technique could lead to clinical detection of the disease" BIOPHOTONICS INTERNATIONAL, LAURIN PUBL., PITTSFIELD, MA, US, vol. 12, no. 4, 1 April 2005 (2005-04-01), pages 60-61, XP008098932 ISSN: 1081-8693

## Beschreibung

Die Erfindung betrifft ein Verfahren zur Bestimmung eines Analyten in einer Probe mittels eines Assays, der im Einschritt-Format ohne Waschschritte durchgeführt werden kann.

Das Immunsystem eines Säugetierorganismus produziert als Antwort auf das Einbringen fremder Substanzen Antikörper, die auch Immunglobuline genannt werden. Sie dienen zur Abwehr der Fremdsubstanzen, die auch als Antigene bezeichnet werden. Die Immunglobuline können in fünf verschiedene Klassen eingeteilt werden. Man unterscheidet Immunglobuline der Klassen M, G, A, E und D. Diese fünf Immunglobulinklassen unterscheiden sich jeweils in der Zusammensetzung der schweren Kette, die als µ-, γ-, α-, ε- bzw. δ-Kette bezeichnet wird.

Jede Immunglobulinklasse hat im Organismus eine andere Aufgabe. Die Immunglobuline der Klasse M treten bei einem ersten Kontakt mit dem Antigen, der so genannten Erstimmunisierung, auf. Die Konzentration dieser Immunglobuline nimmt jedoch bei fortschreitender Infektion schnell wieder ab. Die Immunglobuline der Klasse G werden bei einer ersten Immunisierung erst langsam gebildet und treten bei einer zweiten Infektion mit demselben Antigen in großen Mengen auf. Die Immunglobuline der Klasse A sind auf den Schleimhautoberflächen des Organismus anzutreffen und sind für die dortigen Abwehrvorgänge zuständig. Die Immunglobuline der Klasse E sind hauptsächlich für allergische Reaktionen verantwortlich. Die genaue Funktion der Immunglobuline der Klasse D ist bislang nicht bekannt.

Die einzelnen Immunglobulinklassen kommen im Blut in sehr unterschiedlichen Konzentrationen vor. So sind die Immunglobuline der Klasse G (IgG) in normalem menschlichen Serum mit einem Anteil von etwa 75 %, das entspricht einem Serumgehalt von 8 bis 18 mg/ml, die am stärksten vertretene Klasse. Das am zweithäufigsten auftretende Immunglobulin ist das IgA, dessen durchschnittliche Serumkonzentration 0,9 bis 4,5 mg/ml beträgt. Immunglobuline der Klasse M sind in einer Konzentration von 0,6 bis 2,8 mg/ml, Immunglobuline der Klasse D von 0,03 bis 0,4 mg/ml vorhanden. Am geringsten ist der Anteil von IgE-Antikörpern, die nur in einer Konzentration von 0,02 bis 0,05 µg/ml im Serum auftreten.

Im Stand der Technik werden verschiedene Methoden zum Nachweis von für ein Antigen spezifischen Antikörpern einer bestimmten Klasse beschrieben. So wird der Nachweis von antigenspezifischen Antikörpern einer bestimmten Klasse in einer Probe häufig dadurch geführt, dass die spezifischen Antikörper an eine mit dem spezifischen Antigen beschichtete Festphase gebunden werden. Der Nachweis der für das Antigen spezifischen, nun an die Festphase gebundenen Immunglobuline (Ig) erfolgt durch die Bindung von Antikörpern, die spezifisch gegen humanes Ig einer bestimmten Klasse gerichtet sind, an die nachzuweisenden Ig-Moleküle. Die gegen humanes Ig gerichteten Antikörper sind mit einer Markierung versehen, über die die Detektion stattfindet. Eine solche Testführung (indirektes Testformat) ist aber nur möglich, wenn vor der Reaktion mit den klassenspezifischen, gegen humanes Ig gerichteten markierten Antikörpern durch Waschung alles unspezifische, nicht gebundene Ig entfernt wird. Eine Einschritt-Testführung, wie sie für automatisierte Systeme oft benötigt wird, ist somit in diesem Format nicht möglich.

Eine Möglichkeit, einen Antikörper-Nachweis in einem Einschritt-Test durchzuführen, eröffnet der so genannte Brückentest. Das Brückentest-Konzept ist in der EP-A-0 280 211 beschrieben. Dabei wird ein erster Bindepartner, der mit dem zu bestimmenden Antikörper spezifisch bindefähig ist, wie beispielsweise ein Antigen, an eine Festphase gebunden. Der zu bestimmende Antikörper bindet an das festphasengebundene Antigen. Im Testansatz ist außerdem ein weiteres, spezifisches Antigen vorhanden, das mit einer Markierung versehen ist. Der Nachweis des Antikörpers erfolgt über die Markierung. Sofern in der Probe Immunglobuline verschiedener Klassen, aber gleicher Spezifität vorhanden sind, unterscheidet der Test zwischen den verschiedenen Klassen nicht.

EP 0 944 838 B1 offenbart ein Brückentest-Nachweisverfahren, welches die selektive Bestimmung von antigenspezifischen IgG-Antikörpern in einer Probe ermöglicht, die auch IgM-Antikörper mit der selben Spezifität aufweist. Hierzu wird das Antigen in monomerer Form eingesetzt. Das monomere Antigen bewirkt, dass IgM-Antikörper mit gleicher Spezifität nicht oder nur schwach mit dem Antigen reagieren können.

Spezifische Antikörpernachweise haben eine große Bedeutung in der Infektionsserologie, in der die Immunantwort gegen antigene Strukturen von Krankheitserregern detektiert wird. Obgleich unterschiedliche Quellen zur Gewinnung von Antigenen der Erreger Stand der Technik sind (rekombinante Expression in prokaryontischen und eukaryontischen Systemen; kontrollierte Erregeranzucht, Gewinnung von Antigenen aus natürlichen Quellen) ist es nicht immer möglich, Antigene in monomerer Form zu erhalten, um so einen IgG-selektiven Immunoassay im Einschritt-Format zu führen.

EP 0 957 360 B1 beschreibt die Verwendung von Rheumafaktoren zur Verminderung des Hook-Effektes in Verfahren zur Bestimmung eines Analyten.

EP 1 098 198 A1 beschreibt einen Immunassay zur Bestimmung von humanen IgG-Antikörpern. Hierbei wird ein bestimmter monoklonaler Antikörper eingesetzt, welcher gegen ein Konformationsepitop des Fc-Fragments von menschlichem IgG gebunden an ein Antigen gerichtet ist. Dieser Antikörper wird zur selektiven Erkennung von IgG verwendet und kann zwischen IgG, welche ein Antigen binden, und IgG, welche kein Antigen binden, unterscheiden. Die Gegenwart von ungebundenem IgG interferiert jedoch und führt zu Signaleinbußen.

EP 1 653 233 A1 beschreibt ein Testverfahren zur Bestimmung von antigenspezifischen Antikörpern der Klasse IgG, worin eine Probe mit einer Festphase in Kontakt gebracht wird, an die Antigene gebunden sind. In diesem Verfahren wird ein monoklonaler Antikörper verwendet, welcher zwischen unspezifisch an die Festphase gebundenen Immunglobulinen und an das Antigen gebundenen Immunglobulinen unterscheidet. Nach dem Inkontaktbringen der Probe mit der Festphase wird gewaschen, danach wird der monoklonale Antikörper aufgebracht.

Ein mit Analyseautomaten durchführbarer Immunassay zum Nachweis von Antigen-spezifischem Immunglobulin G im Einschritt-Format wird in der Patentanmeldung WO 99/15898 (Chien et al.) beschrieben. Dort wird ein Antikörper verwendet, der spezifisch für den konstanten Teil des humanen Immunglobulins G ist. In diesem Assay sind die Interferenzen durch freies, unspezifisches IgG ohne ausreichende Diskriminierung von freiem und komplexiertem IgG signifikant.

Yin et al. (Anal. Chem. 2005,77:3525-3530) beschreibt einen Sandwich-Immuntest zum zum Nachweise von IgG.

Klaassen et al. (British Journal of Haematology, 1991:77:398-402) offenbart ein Verfahren zur Bestimmung eines antigenspezifischen Antikörpers der Immunglobulinklasse G. Nachgewiesen wird ein bivalenter Immunkomplex mit einem festphasengebundenen Rezeptor (C1q) und einem zweiten Rezeptor (125|-markierten Antikörper).

Boas (Biophotonics International, April 2005, 60-61) offenbart einen Sandwich-Immuntest zum Nachweis von Amyloid-abgeleiteten diffusiblen Liganden mithilfe eines Antikörpers, welcher für Amyloid-β-Oligomere spezifisch ist.

Einschritt-Verfahren des Standes der Technik zum Nachweis von IgG können also nicht ausreichend zwischen freiem und gebundenem IgG unterscheiden. Um eine Diskriminierung zwischen IgG und IgM gleicher Spezifität zu erzielen, müssen Antigene monomer sein, eine Forderung, die oft nicht erfüllbar ist.

Aufgabe der vorliegenden Erfindung war es daher, ein Verfahren zum Nachweis eines Analyten in einer Probe, insbesondere von antigenspezifischen Antikörpern der Immunglobulinklasse G, zu entwickeln, welches im Einschritt-Verfahren durchgeführt werden kann, um vorteilhaft in automatisierten Systemen angewendet werden zu können. Außerdem hat die vorliegende Erfindung die Aufgabe, die Nachteile des Standes der Technik zumindest teilweise zu überwinden. Hierzu ist eine ausreichende Diskriminierung zwischen freiem Analyten und gebundenem Analyten notwendig.

In einem ersten Aspekt betrifft die vorliegende Erfindung ein Verfahren zur Bestimmung eines Analyten in einer Probe, umfassend die Schritte:
(a) Inkontaktbringen der Probe mit einem ersten analytspezifischen Rezeptor und einem zweiten analytspezifischen Rezeptor, wobei einer der beiden Rezeptoren an eine Festphase gebunden ist oder an eine Festphase bindefähig ist und der andere Rezeptor eine signalgebende Gruppe trägt oder mit einer signalgebenden Gruppe bindefähig ist, und
(b) Nachweisen des Vorhandenseins oder/und der Menge des Analyten durch Bestimmung der signalgebenden Gruppe auf der Festphase,
dadurch gekennzeichnet, dass der erste analytspezifische Rezeptor mindestens zwei Bindestellen für den Analyten enthält und der zweite analytspezifische Rezeptor an eine Anordnung aus mindestens zwei Analytmolekülen, welche an den ersten Rezeptor gebunden sind, bindet und diese Anordnung von einem einzelnen Analytmolekül unterscheiden kann und der Analyt ein antigenspezifischer Antikörper ist.

Überraschenderweise wurde gefunden, dass das erfindungsgemäße Verfahren nicht durch die Anwesenheit von Rheumafaktor in einer Probe gestört wird.

Der Analyt ist ein antigenspezifischer Antikörper, z.B. ein antigenspezifischer Antikörper der Klasse G, M, A, D oder/und E. Stärker bevorzugt ist der Analyt ein antigenspezifischer Antikörper der Klasse G, z.B. gegen ein Pathogen, ein Tumorantigen oder/und ein Autoantigen.

Die Probe umfassend den Analyten ist vorzugsweise eine flüssige Probe. Die flüssige Probe kann eine beliebige flüssige Probe sein, z.B. eine wässrige Probe. Die Probe kann eine biologische Probe oder/und eine klinische Probe wie z.B. eine Körperflüssigkeit sein, z.B. Blut, Serum, Urin, Sputum, Eiter, etc. Stärker bevorzugt kann eine Probe Rheumafaktor enthalten.

In einer bevorzugten Ausführungsform sind der erste und der zweite analytspezifische Rezeptor voneinander verschieden.

Im erfindungsgemäßen Verfahren ist der zweite Rezeptor selektiv bindefähig an eine Anordnung aus mindestens zwei Analytmolekülen, welche an den ersten Rezeptor gebunden sind. Unter "einer Anordnung aus mindestens zwei Analytmolekülen" wird verstanden, dass die mindestens zwei Analytmoleküle zusammen eine Struktur aufweisen, an die der zweite Rezeptor selektiv bindefähig ist, d.h. die von dem zweiten Rezeptor erkannt werden kann und die der zweite Rezeptor von einem einzelnen Analytmolekül unterscheiden kann, insbesondere von einem einzelnen Analytmolekül, welches nicht an den ersten Rezeptor gebunden ist. Unter "selektiv bindefähig" wird auch verstanden, dass der zweite Rezeptor an die Anordnung aus mindestens zwei Analytmolekülen stärker binden kann als an ein einzelnes Analytmolekül. Eine Anordnung kann auch ein Immunkomplex sein, umfassend mindestens zwei Analytmoleküle, insbesondere Antikörper, welche an den ersten Rezeptor gebunden sind. Der erste Rezeptor kann ein Antigen mit mindestens zwei Epitopen umfassen.

Vorzugsweise enthält eine "Anordnung" mindestens zwei an den ersten Rezeptor gebundene Analytmoleküle, die miteinander "aggregiert" sind. ,,Aggregiert" oder "Aggregat" im Sinne der vorliegenden Erfindung bedeutet, dass die an den Rezeptor gebundenen Analytmoleküle einen bestimmten Abstand zueinander haben, welcher so gewählt wird, dass der zweite analytspezifische Rezeptor an die mindestens zwei an den ersten Rezeptor gebundenen Analytmoleküle bindet. Die an den ersten Rezeptor gebundenen Analytmoleküle befinden sich also in räumlicher Nähe zueinander. Die Abstände der an den ersten Rezeptor gebundenen Analytmoleküle werden durch den Abstand der Bindestellen im Rezeptor bestimmt. ,,Aggregiert" oder ,,Aggreat" in Sinne der vorliegenden Erfindung umfasst, dass die an den ersten Rezeptor gebundenen Analytmoleküle untereinander nicht chemisch gebunden sind oder untereinander chemische Bindungen aufweisen, z.B. kovalente Bindungen oder nicht-kovalente Bindungen, wie z.B. Wasserstoffbrückenbindungen oder Antigen-Antikörperbindungen.

Der erste analytspezifische Rezeptor enthält mindestens zwei Bindungsstellen für den Analyten. Der erste analytspezifische Rezeptor kann mindestens zwei verschiedene Bindungsstellen, mindestens zwei gleiche Bindungsstellen oder eine Kombination von verschiedenen und gleichen Analytbindungsstellen enthalten.

In noch einer weiteren bevorzugten Ausführungsform ist der erste Rezeptor ein oligomerer oder ein multimerer Rezeptor. "Oligomer" oder ,,multimer" im Sinne der vorliegenden Erfindung bedeutet, dass der erste Rezeptor mehrere Bindestellen für den Analyten enthält, vorzugsweise wenigstens 3, wenigstens 4, wenigstens 5, wenigstens 10, wenigstens 20, wenigstens 50 oder wenigstens 100 Bindestellen. Der erste Rezeptor enthält vorzugsweise bis zu 200, bis zu 100, bis zu 50, bis zu 20, bis zu 10 oder bis zu 5 Bindestellen. "Oligomer" oder ,,multimer" im Sinne der vorliegenden Erfindung schließt auch ein, dass der erste Rezeptor ein Oligomer oder ein Multimer aus Bausteinen sein kann, wobei jeder Bausteine jeweils keine, eine oder mehrere Bindestellen aufweisen kann. "Oligomer" oder ,multimer" umfasst auch ,,oligovalent" oder "multivalent".

In einer weiteren bevorzugten Ausführungsform wird der erste Rezeptor in Form von Rezeptorvorläufern bereitgestellt. Vorzugsweise werden mindestens zwei Rezeptorvorläufer bereitgestellt. Vorzugsweise assoziieren die Rezeptorvorläufer miteinander oder binden aneinander, um den ersten Rezeptor zu bilden. Die Rezeptorvorläufer können derartig beschaffen sein, dass sie während des Schrittes (a) des erfindungsgemäßen Verfahrens assoziieren oder aneinander binden können, um den ersten Rezeptor zu bilden. Sie können auch derartig beschaffen sein, dass sie vor dem Schritt (a) des erfindungsgemäßen Verfahrens assoziieren oder aneinander binden können. Die mindestens zwei Rezeptorvorläufer können so beschaffen sein, dass entweder einer der Rezeptorvorläufer alle Bindestellen für den Analyten umfasst, oder dass beide Rezeptorvorläufer mindestens eine Bindestelle für den Analyten umfassen.

Die mindestens zwei Rezeptorvorläufer können durch eine chemische Bindung miteinander assoziieren oder aneinander binden, wobei die Bindung kovalent oder nicht-kovalent sein kann. Zur Assoziation oder Bindung kann jedes beliebige Bindungssystem eingesetzt werden, welches dem Fachmann bekannt ist. Bevorzugt sind hierin beschriebene X/anti-X-Bindungssysteme wie z.B. das Avidin/Streptavidin-Biotinsystem. So kann einer der mindestens zwei Rezeptorvorläufer eine Biotingruppe enthalten, und der andere Rezeptorvorläufer enthält eine Avidin- oder Streptavidingruppe. Beispielsweise kann ein biotinyliertes Molekül, z.B. ein Peptid, welches mindestens eine Bindestelle für den Analyten trägt, über die Bindung bzw. Assoziation an ein mit Avidin oder Streptavidin beschichtetes Bead einen ersten Rezeptor bilden, welcher mindestens zwei Bindestellen für den Analyten umfasst. Ebenso kann der Bead biotinyliert sein und das Molekül, welches mindestens eine Bindegruppe trägt, kann mit Avidin oder Streptavidin gekoppelt sein.

Der erste Rezeptor kann also einen Bead umfassen, welcher eine Anordnung von mindestens zwei Bindestellen für den Analyten trägt. Es ist bevorzugt, dass der erste Rezeptor einen Bead umfasst, welcher mindestens 10, mindestens 50 oder mindestens 100 Bindestellen umfasst.

In noch einer weiteren bevorzugten Ausführungsform des erfindungsgemäßen Verfahrens umfasst der erste Rezeptor ein Antigen, welches mindestens zwei Epitope als Bindestellen für den Analyten enthält. In dieser Ausführungsform ist der Analyt ein Antikörper, bevorzugt ein Antikörper der Klasse G, M, A, D oder/und E, noch stärker bevorzugt ein Antikörper der Klasse G.

Das erfindungsgemäße Antigen kann ein oligo- oder multimeres Antigen sein. Die mindestens zwei Epitope können gleich oder verschieden sein. Das Antigen kann mindestens 2 verschiedene Epitope, mindestens 2 gleiche Epitope oder eine Kombination von gleichen und verschiedenen Epitopen umfassen. Das Antigen kann ein repetitives Epitop umfassen. Das Antigen kann mehrere Epitope enthalten, vorzugsweise wenigstens 3, wenigstens 4, wenigstens 5, wenigstens 10, wenigstens 20, oder wenigstens 50 Epitope. Das Antigen kann bis zu 100, bis zu 50, bis zu 20, bis zu 10 oder bis zu 5 Epitope enthalten.

Das erfindungsgemäße Antigen kann beliebige Epitope mit unterschiedlichen strukturellen Merkmalen und Zusammensetzungen aufweisen. Das Antigen kann einen Mikroorganismus, eine Zelle, eine Zellorganelle, ein Zellkompartiment oder ein Lysat oder eine Präparation davon umfassen, z.B. eine Membranpräparation. Das Antigen kann ein aus einer natürlichen Quelle isoliertes Antigen sein oder durch rekombinante Expression in prokaryontischen oder/und eukaryontischen Organismen oder/und durch chemische Synthese hergestellt werden. Gegebenenfalls kann die Oligo- oder Multimerisierung des Antigens durch natürliche Assemblierung oder Aggregation oder durch gezielte adsorptive oder kovalente Verknüpfung oder durch adsorptive oder kovalente Kopplung herbeigeführt werden.

In einer weiteren bevorzugten Ausführungsform umfasst der zweite analytspezifische Rezeptor einen Antikörper. Stärker bevorzugt sind Antikörper mit Rheumafaktor-ähnlichen Eigenschaften, die bevorzugt aggregierte oder oligomerisierte Immunglobuline, jedoch keine singulären Immunglobuline binden können.

Ebenso bevorzugt umfasst der zweite Rezeptor einen Antikörper mit niedriger Affinität für die Bindung des Analyten. Unter der Affinität eines erfindungsgemäßen Antikörpers wird die Stärke aller nicht-kovalenten Interaktionen zwischen der einzelnen Antigen-Bindungsstelle auf einem Antikörper und dem einzelnen Epitop definiert. Antikörper mit niedriger Affinität binden schwach und dissoziieren rasch, während hochaffine Antikörper stärker binden und länger gebunden bleiben.

Die Affinität an einer Bindungsstelle reflektiert nicht immer die wirkliche Stärke einer Antigen-Antikörper Interaktion, wie z.B. zwischen den erfindungsgemäßen Antigenen als erste Rezeptoren mit mindestens zwei Epitopen, an welche Analytmoleküle, z.B. antigenspezifische Antikörper gebunden sind, und z.B. Antikörpern mit mehreren Bindungsstellen als zweite Rezeptoren. Die Interaktion von Antigen und einer Antigen-Bindungsstelle eines Antikörpers an einer Stelle steigert die Wahrscheinlichkeit zur Reaktion an einer zweiten Antigen-Bindungsstelle desselben Antikörpers, dadurch kann eine Vernetzung der Interaktionspartner entstehen. Die Stärke von solchen mehrfachen Interaktionen zwischen multivalentem Antikörper und Antigen wird als Avidität bezeichnet. Dabei kompensiert eine hohe Avidität eine niedrige Affinität wie z.B. beim pentameren Immunglobulin IgM.

Der zweite Rezeptor ist in einer bevorzugten Ausführungsform ein Antikörper, welcher mindestens zwei, mindestens drei, mindestens vier, mindestens fünf und besonders bevorzugt 10 oder mehr Paratope umfasst.

In der vorliegenden Erfindung umfasst der zweite Rezeptor stärker bevorzugt einen Antikörper mit niedriger Affinität und hoher Avidität für den Analyten, z.B. einen Antigen-spezifischen Antikörper, umfassend mindestens zwei, mindestens drei, mindestens vier, mindestens fünf und besonders bevorzugt 10 oder mehr Paratope. Ein noch stärker bevorzugter zweiter Rezeptor ist ein antigenspezifischer Antikörper der Immunglobulinklasse IgM oder untereinander vernetzte IgG-Immunglobuline.

Am stärksten bevorzugt ist der Antikörper <h-agg.IgG>IgM.

Vorzugsweise weisen die erfindungsgemäßen niedrigaffinen Antikörper einen Affinitätskoeffizienten von größer als 10⁻⁸ M, vorzugsweise größer als 10⁻⁷ M, stärker bevorzugt größer als 10⁻⁶ M auf.

Der erfindungsgemäße niedrigaffine Antikörper als zweiter Rezeptor erkennt Antigen-spezifische Antikörper, welche in einer Anordnung oder/und als Aggregat an dem ersten Rezeptor gebunden sind, z.B. in ausreichender Dichte. Ungebundene oder unspezifisch auf dem ersten Rezeptor gebundene Analytmoleküle, die locker und ungleichmäßig verteilt sind, werden vorzugsweise nicht oder vernachlässigbar erkannt. Damit ist ein solcher erfindungsgemäßer zweiter analytspezifischer Rezeptor selektiv bindefähig an einen ersten Rezeptor mit mindestens zwei daran gebundenen Analytmolekülen.

Antikörper der Klasse IgM haben die Eigenschaften der allgemeinen Klasse rheumatoider Antikörper, d.h. sie binden bevorzugt die Antigen-spezifisch gebundenen Antikörper der Immunglobulinklasse IgG, da sie nur die ausreichend dicht gepackten Antigen-spezifischen Antikörper auf dem multimeren Antigen des Immunoassays erkennen. Nicht komplexierte, freie Immunglobuline G, die insbesondere nicht spezifisch für das Antigen sind, werden nicht oder nur vernachlässigbar erkannt.

Auf diese Weise gelingt zum Beispiel der Nachweis von Antigen-spezifischen IgG in Gegenwart von Immunglobulinen anderer Klassen mit gleicher Antigenspezifität wie auch in Gegenwart von unspezifischen IgG ohne für den Assay schädlichen Sensitivitätsverlust. Der Einsatz des erfindungsgemäßen zweiten Rezeptors erreicht eine hohe Diskriminierung zwischen Antigen-spezifisch gebundenem Immunglobulin G und nicht antigen-spezifischem, freiem IgG. Weiterhin erkennt der erfindungsgemäße zweite Rezeptor bevorzugt die auf dem erfindungsgemäßen Antigen spezifisch gebundenen, benachbart lokalisierten Immunglobulinmoleküle, welche oligomerisiert sein können, aber nicht oder vernachlässigbar gering freie, unkomplexierte antigenspezifische Immunglobuline.

Somit ist der Nachweis Antigen-spezifischer Immunglobuline der Klasse G in einer Probe in Gegenwart der natürlich vorkommenden, im Überschuss vorliegenden nicht antigen-spezifischen IgG-Moleküle im Einschritt-Format gewährleistet.

Unter "Einschritt-Verfahren" wird verstanden, dass die Reaktion des Analyten mit den erfindungsgemäßen Reaktanten in einem Reaktionsansatz durchgeführt werden kann, insbesondere ohne dass Komponenten des Ansatzes durch einen oder mehrere Waschschritte voneinander abgetrennt werden müssen.

"Einschritt-Vertahren" oder "Reaktion in einem Reaktionsansatz" schließt ein, dass die Reaktanten gleichzeitig/nacheinander zu der Reaktionsmischung (Probe) hinzugefügt werden können, oder/und dass weitere Hilfsstoffe, (z.B. Puffer, Salze, etc.) gleichzeitig/nacheinander hinzugefügt werden können.

Ein bevorzugtes ,,Einschritt-Verfahren" wird ohne einen Waschschritt durchgeführt.

In einer weiteren bevorzugten Ausführungsform wird im erfindungsgemäßen Verfahren Schritt (a) in einem Reaktionsansatz ohne Waschschritt durchgeführt. Stärker bevorzugt werden die Schritte (a) und (b) in einem Reaktionsansatz ohne Waschschritt durchgeführt. Das erfindungsgemäße Verfahren ermöglicht den Nachweis antigenspezifischer Antikörper der Immunglobulinklasse G im Einschritt-Format. Diese Antikörpernachweise sind von besonderer Bedeutung in der in-vitro Diagnostik von Infektionskrankheiten sowie Autoimmunerkrankungen.

In anderen Ausführungsformen kann das erfindungsgemäße Verfahren einen Waschschritt umfassen.

In einer am meisten bevorzugten Ausführungsform ist im erfindungsgemäßen Verfahren der erste Rezeptor ein erfindungsgemäßes Antigen, der zweite Rezeptor ist ein Rheumafaktor-ähnlicher Antikörper, insbesondere ein Immunglobulin der Klasse M, und der Analyt ist ein antigenspezifisches Immunglobulin G. So ist die Bindung mehrerer spezifischer Immunglobuline G an den Antigen-Bindepartner möglich, was wiederum eine Oligomerisierung der IgG-Moleküle zur Folge haben kann, d.h. zwei oder mehr IgGs können sich in räumlicher Nähe zueinander befinden und werden dadurch für den Rheumafaktor-ähnlichen Rezeptor bindefähig.

In noch einer weiteren bevorzugten Ausführungsform ist das erfindungsgemäße Verfahren ein diagnostisches Verfahren, insbesondere zum Nachweis von Antikörpern in biologischen Proben, insbesondere klinischen Proben. Im erfindungsgemäßen diagnostischen Verfahren können z.B. Antikörper gegen ein Pathogen, z B. ein Virus oder einen Mikroorganismus, gegen ein Tumorantigen oder/und Autoantikörper nachgewiesen werden. Insbesondere können Antikörper gegen Viren wie Rubella, CMV, HAV, HBV, HCV nachgewiesen werden.

Im erfindungsgemäßen Verfahren können als Rezeptoren auch Antikörper-Fragmente eingesetzt werden. Die Fragmentierung der Antikörper ist dem Fachmann bekannt und erfolgt nach konventionellen Techniken. Beispiele für proteolytisch gespaltene und/oder rekombinant hergestellte Fragmente beinhalten Fab, F(ab')2, Fab', Fv und Einzelstrang-Antikörper (scFv), die eine V[L] und/oder V[H] Domäne mit einem Peptid-Linker enthalten. Die scFv's können kovalent oder nicht kovalent verbunden sein, so dass ein Antikörper mit zwei oder mehr Bindungsstellen entsteht. Der erfindungsgemäße Antikörper kann ein polyklonaler, monoklonaler, chimärer oder humanisierter Antikörper sein, welcher rekombinant hergestellt sein kann. Es ist bevorzugt, dass der zweite Rezeptor ein monoklonaler Antikörper ist.

Der erfindungsgemäße Antikörper ist bevorzugt ein monoklonaler Antikörper insbesondere der Klasse IgM mit der Eigenschaft einer niedrigen Affinität und einer hohen Avidität für IgG, insbesondere humanes IgG. Dieser Antikörper kann über eine Mehrpunktbindung die Erkennung von humanem IgG in komplexiertem Zustand mittels einer hohen Avidität selektiv gewährleisten.

Einer der beiden Rezeptoren ist erfindungsgemäß an eine Festphase gebunden oder an eine Festphase bindefähig, und der andere der beiden Rezeptoren trägt eine signalgebende Gruppe oder ist mit einer signalgebenden Gruppe bindefähig. In einer bevorzugten Ausführungsform ist der erste Rezeptor an die Festphase gebunden oder an die Festphase bindefähig, und der zweite Rezeptor trägt die signalgebende Gruppe oder ist an die signalgebende Gruppe bindefähig. In einer anderen bevorzugten Ausführungsform ist der zweite Rezeptor an die Festphase gebunden oder an die Festphase bindefähig, und der erste Rezeptor trägt die signalgebende Gruppe oder ist an die signalgebende Gruppe bindefähig.

Als signalgebende Gruppe können alle dem Fachmann geläufigen signalgebenden Gruppen Verwendung finden. Bevorzugt wird eine direkt nachweisbare Gruppe, beispielsweise eine chemilumineszierende, fluoreszierende oder radioaktive Gruppe oder ein Metallsol-, Latex- oder Goldpartikel eingesetzt. Der Nachweis der signalgebenden Gruppen kann auf dem Fachmann bekannte Weise erfolgen.

Der bindefähige erste oder zweite Rezeptor ist vorzugsweise löslich und trägt vorzugsweise eine Markierung, welche dazu dient, den Rezeptor zu binden. Bevorzugt ist als Markierung ein Partner eines X/anti-X-Bindungssystems, z.B. ein Partner einer Kombination ausgewählt aus Biotin/Avidin, Biotin/Streptavidin, Biotin/Antibiotin, Hapten/Anti-Hapten, Fc-Fragment eines Antikörpers und Antikörper gegen dieses Fc-Fragment, ein Anti-Antikörper oder Kohlenhydrat und Lectin. Der andere Reaktionspartner des Bindungssystems ist an den Träger bzw. die signalgebende Gruppe gebunden. Die Bindung der Partner des Bindungssystems kann nach den üblichen, dem Fachmann bekannten Methoden vorgenommen werden. Hierbei ist sowohl eine kovalente als auch eine adsorptive Bindung geeignet.

In einer weiteren Ausführungsform ist die Markierung eine signalgebende Gruppe, wie sie hierin beschrieben ist. Bevorzugt wird als Markierung eine direkt nachweisbare Gruppe verwendet. Die Verfahren zur Markierung des Rezeptors sind als Stand der Technik bekannt. Der Nachweis der Markierung erfolgt in an sich bekannter Weise direkt durch Messung signalgebender Gruppe.

Der Nachweis der Markierung kann auch auf indirekte Weise erfolgen. Dabei bindet ein weiterer Bindepartner, der selbst wiederum mit einer signalgebenden Gruppe gekoppelt ist, spezifisch an den Rezeptor, an eine Markierung am Rezeptor, an das Antigen oder/und an eine Markierung am Antigen.

Eine am meisten bevorzugte Ausführungsform des erfindungsgemäßen Verfahrens ist ein Verfahren zum Nachweis von Antigen-spezifischen Antikörpern der Immunglobulinklasse G in einer Probe mittels eines Immunoassays im Einschritt-Format, umfassend die Schritte:
(a) Inkontaktbringen einer Probe, welche Antikörper der Immunglobulinklasse G enthält, die spezifisch für ein Antigen sind, mit einem ersten Rezeptor, der ein Antigen in oligomerer oder multimerer Form umfasst und eine signalgebende Gruppe trägt, und mit einem zweiten Rezeptor, der Antigen-gebundenes, aggregiertes Immunglobulin G selektiv bindet und eine Markierung trägt, unter Bedingungen, unter denen sich ein Immunkomplex aus Antigen, daran spezifisch gebundenen Immunglobulinen G und dem zweiten Rezeptor bildet;
(b) Inkontaktbringen des Reaktionsgemisches aus (a) mit einer festen Phase, welche einen Bindepartner für die Markierung des zweiten Rezeptors immobilisiert enthält, unter Bedingungen, unter denen der Immunkomplex aus (a) über den zweiten Rezeptor an die Festphase bindet;
(c) Nachweis des gebundenen Komplexes aus (b) über die signalgebende Gruppe.

Eine weitere am meisten bevorzugte Ausführungsform des erfindungsgemäßen Verfahrens ist ein Verfahren zum Nachweis von Antigen-spezifischen Antikörpern der Immunglobulinklasse G in einer Probe mittels eines Immunoassays im Einschritt-Format, umfassend die Schritte:
(a) Inkontaktbringen einer Probe, welche Antikörper der Immunglobulinklasse G enthält, die spezifisch für ein Antigen sind, mit einem ersten Rezeptor, der ein Antigen in oligomerer oder multimerer Form umfasst, wobei der erste Rezeptor eine Markierung trägt, und mit einem zweiten Rezeptor, der Antigen-gebundenes, aggregiertes Immunglobulin G selektiv bindet und eine signalgebende Gruppe trägt, unter Bedingungen, unter denen sich ein Immunkomplex aus Antigen, daran spezifisch gebundenen Immunglobulinen G und dem zweiten Rezeptor bildet;
(b) Inkontaktbringen des Reaktionsgemisches aus (a) mit einer festen Phase, welche einen Bindepartner für die Markierung des ersten Rezeptors immobilisiert enthält, unter Bedingungen, unter denen der Immunkomplex aus (a) über den ersten Rezeptor an die Festphase bindet;
(c) Nachweis des gebundenen Komplexes aus (b) über die signalgebende Gruppe.

Ein weiterer erfindungsgemäßer Gegenstand ist ein Kit, welcher zur Verwendung in dem hierin beschriebenen erfindungsgemäßen Verfahren einschließlich der bevorzugten Ausführungsformen geeignet ist.

Bevorzugt ist ein Kit, welcher einen ersten analytspezifischen Rezeptor und einen zweiten analytspezifischen Rezeptor umfasst, wobei einer der beiden Rezeptoren an eine Festphase gebunden ist oder an eine Festphase bindefähig ist und der andere Rezeptor eine signalgebende Gruppe trägt oder mit einer signalgebenden Gruppe bindefähig ist, dadurch gekennzeichnet, dass der erste analytspezifische Rezeptor mindestens zwei Bindestellen für den Analyten enthält und der zweite analytspezifische Rezeptor an eine Anordnung aus mindestens zwei Analytmolekülen, welche an den ersten Rezeptor gebunden sind, bindet und diese Anordnung von einem einzelnen Analytmolekül unterscheiden kann und der Analyt ein antigenspezifischer Antikörper ist.

Stärker bevorzugt wird ein erfindungsgemäßer Kit, wobei der erste analytspezifische Rezeptor und der zweite analytspezifische Rezeptor zur Anwendung in einem Reaktionsansatz formuliert werden.

Eine stärker bevorzugte Ausführungsform betrifft einen Kit, wobei der erste Rezeptor an die Festphase gebunden ist oder an die Festphase bindefähig ist und der zweite Rezeptor die signalgebende Gruppe trägt oder an die signalgebende Gruppe bindefähig ist.

Eine andere ebenfalls stärker bevorzugte Ausführungsform betrifft einen Kit, wobei der zweite Rezeptor an die Festphase gebunden ist oder an die Festphase bindefähig ist und der erste Rezeptor die signalgebende Gruppe trägt oder an die signalgebende Gruppe bindefähig ist.

In einer weiteren bevorzugten Ausführungsform kann der erste Rezeptor im erfindungsgemäßen Kit auch in Form von Rezeptorvorläufern bereitgestellt werden. Die Rezeptorvorläufer sind hierin in Zusammenhang mit dem erfindungsgemäßen Verfahren beschrieben. Insbesondere kann der Kit als Rezeptorvorläufer einen Bead und einen weiteren Rezeptorvorläufer, z.B. ein Peptid, enthalten.

Der erfindungsgemäße Kit kann als ersten Rezeptor auch einen Bead umfassen, welcher mindestens 10, mindestens 50 oder mindestens 100 Bindestellen umfasst.

Ein weiterer erfindungsgemäßer Aspekt betrifft die Verwendung eines analytspezifischen Rezeptors, welcher mindestens zwei Bindestellen für den Analyten enthält, in einem Verfahren zur Bestimmung eines Analyten in einer Probe, welches hierin beschrieben ist. Stärker bevorzugt ist dieses Verfahren ein hierin beschriebenes diagnostisches Verfahren.

Die Erfindung wird durch die folgenden Beispiele erläutert. Jedes der Beispiele enthält erfindungsgemäße Daten und Vergleichsdaten, welche mit Verfahren des Standes der Technik gewonnen wurden.

### Beispiele

### Beispiel 1: Nachweis von Anti-Rubella spezifischem Immunglobulin G in Humanseren nach dem erfindungsgemäßen Verfahren und Vergleich der Sensitivität des erfindungsgemäßen Verfahrens mit einem Immunoassay des Standes der Technik im indirekten Testformates mit Waschschritt.

### 1.1 Erfindungsgemäßes Verfahren

Eine immunologische Detektion von Anti-Rubella-IgG in nativen Sera wurde mit einem automatierten Elecsys® 2010-Analyzer (Roche Diagnostics GmbH) durchgeführt. Messungen wurden unter Verwendung eines erfindungsgemäßen Antikörpers als Capture-Antikörper ausgeführt. Das Biotin-Konjugat dieses Antikörpers wurde auf der Oberfläche von Streptavidin-beschichteten magnetischen Beads immobilisiert und bindet daher Anti-Rubella-IgG gebunden an ein multimeres Rubella-like-Partikel (RLP) aus einer Probe. Der Komplex wurde durch einen ruthenylierten monoklonalen Antikörper detektiert, welcher an die RLPs band. Die

Signaldetektion im Elecsys® 2010 basierte auf Elektrochemolumineszenz.

In Anwesenheit eines spezifischen Immunoglobulin-Analyten wurde der chromogene Rutheniumkomplex an die Festphase gebunden und emittierte Licht bei 620 nm nach Anregung an einer Platinelektrode. Das Lichtsignal ist in willkürlichen Einheiten angegeben. Die Messung wurde mit anti-Rubella-IgG-positiven Proben aus einem Panel mit gemischtem Titer und zwei Serokonversionspanelen durchgeführt.

### 1.2. Stand der Technik

Ein Zweischritt-Immunassay (Cobas Core, Rubella IgG recomb II), des Standes der Technik wurde zum Vergleich durchgeführt.

### Panele mit gemischtem Titer

| | Erfindungsgemäßer Einschritt-Immunassay coi<1 neg coi≥1 pos | | Zweischritt-Immunassay <10 U/ml neg ≥10 IU/ml pos |
|---|---|---|---|
| Schwelle | 3500 Counts | | 10 IU/ml |
| Probe | Counts | COI | IU/ml |
| PTR 201-1 | 3867 | 1.1 | 20.8 |
| PTR 201-2 | 73943 | 21.1 | 144.8 |
| PTR 201-3 | 626833 | 179.1 | 134.8 |
| PTR 201-4 | 434690 | 124.2 | 300 |
| PTR 201-5 | 2268 | 0.65 | 0.4 |
| PTR 201-6 | 5371 | 1.53 | 19.6 |
| PTR 201-7 | 185186 | 52.9 | 15.4 |
| PTR 201-8 | 9367 | 2.68 | 26.4 |
| PTR 201-9 | 174982 | 50.0 | 129.1 |
| PTR 201-10 | 134451 | 38.4 | 84.5 |
| PTR 201-11 | 71459 | 20.4 | 56 |
| PTR 201-12 | 582819 | 166.5 | 300 |
| PTR 20113 | 582257 | 166.4 | 109.1 |
| PTR 201-14 | 31982 | 9.1 | 22.2 |
| PTR 201-15 | 645934 | 184.6 | 300 |
| PTR 201-16 | 2250 | 0.64 | 1.2 |
| PTR 201-17 | 36001 | 10.3 | 64.9 |
| PTR 201-18 | 12711 | 3.6 | 16.5 |
| PTR 201-19 | 141224 | 40.3 | 12.1 |
| PTR 201-20 | 350990 | 100.3 | 155.3 |
| PTR 201-21 | 57148 | 16.3 | 91.1 |
| PTR 201-22 | 5030 | 1.4 | 28.7 |
| PTR 201-23 | 30672 | 8.8 | 66.6 |
| PTR 201-24 | 27950 | 8.0 | 8.3 |
| PTR 201-25 | 194719 | 55.6 | 142.5 |

### Serokonversionspanele

| | | Erfindungsgemäßer Einschritt-Immunassay coi<1 neg coi≥1 pos | | Zweischritt-Immunassay <10 U/ml neg ≥ 10 IU/ml pos |
|---|---|---|---|---|
| Probe | Schwelle | 3500 Counts | | 10 IU/ml |
| | Tag | Counts | COI | IU/ml |
| RP 001-001 | 0 | 1767 | 0.50 | 0.8 |
| RP 001-002 | 2 | 1821 | 0.52 | 0.7 |
| RP 001-003 | 7 | 1833 | 0.52 | 0.4 |
| RP 001-004 | 9 | 1732 | 0.49 | 0.6 |
| RP 001-005 | 14 | 1880 | 0.47 | 0.5 |
| RP 001-006 | 17 | 2592 | 0.74 | 0.5 |
| RP 001-007 | 21 | 30305 | 8.66 | 3 |
| RP 001-008 | 24 | 76058 | 21.73 | 5.7 |
| RP 001-009 | 28 | 203264 | 58.08 | 13.8 |
| RP 001-010 | 31 | 293043 | 83.73 | 23.7 |
| RP 001-011 | 35 | 262759 | 75.07 | 23.8 |
| RP 001-012 | 38 | 194288 | 55.51 | 16.3 |
| RP 001-013 | 42 | 181673 | 51.91 | 16.3 |
| RP 001-014 | 45 | 160403 | 45.83 | 14.4 |
| RP 001-015 | 50 | 139584 | 39.88 | 18.1 |
| RP 011-001 | 0 | 1902 | 0.54 | 0 |
| RP 011-002 | 3 | 1766 | 0.50 | 0.2 |
| RP 011-003 | 9 | 1753 | 0.50 | 0 |
| RP 011-004 | 12 | 1720 | 0.49 | 0.1 |
| RP 011-005 | 18 | 3928 | 1.12 | 0.4 |
| RP 011-006 | 19 | 92242 | 26.35 | 7.7 |
| RP 011-007 | 24 | 366359 | 104.57 | 28.8 |
| RP 011-008 | 27 | 408392 | 116.88 | 37.4 |
| RP 011-009 | 31 | 405799 | 115.94 | 33.9 |
| RP 011-010 | 36 | 417007 | 119.14 | 39.1 |
| RP 011-011 | 39 | 414958 | 118.55 | 38 |
| RP 011-012 | 43 | 385813 | 110.23 | 44.4 |
| RP011-013 | 46 | 398357 | 113.82 | 52.8 |

Bei den Serokonversionspanelen konnten positive Titer im Einschrittverfahren am 21. bzw. 18. Tag und im Zweischrittverfahren erst am 28. bzw. 24. Tag nachgewiesen werden.

Das erfindungsgemäße Immunassay-Format ist empfindlicher als der Zweischritt-Immunassay des Standes der Technik, da eine Detektion eines positiven Titers zu einem früheren Zeitpunkt möglich ist.

### Beispiel 2: Vergleich der Performance eines Anti-Human-IgG Antikörper-Rezeptors mit erfindungsgemäßen Eigenschaften (niedrige Affinität, selektive Bindung von aggregiertem Human-IgG) mit einem Anti-Human-IgG Antikörper-Rezeptors mit Eigenschaften des Standes der Technik (hohe Affinität, gleich starke Bindung von aggregiertem und nicht aggregiertem IgG).

Master Calibrators (MC), negative und positive Seren wurden auf Anti-Rubella-IgG-Antikörper mit dem immunologischen Verfahren nach Beispiel 1 untersucht.

| | Mab<h-IgG> IgM-Typ niedrige Affinität coi<1 neg coi≥1 pos | | Mab<h-IgG> IgG-Typ hohe Affinität coi<1 neg coi≥1 pos | |
|---|---|---|---|---|
| Probe | 6859 Counts | | 3521 Counts | |
| | Counts | COI | Counts | COI |
| MC10 IU/ml | 2399 | 0.35 | 1483 | 0.42 |
| MC2 10 IU/ml | 6859 | 1.00 | 3521 | 1.00 |
| MC3 50 IU/ml | 35617 | 5.19 | 3385 | 0.96 |
| MC4 150 IU/ml | 170048 | 24.79 | 6027 | 1.71 |
| MCS 300 IU/ml | 334483 | 48.76 | 10946 | 3.11 |
| | | | | |
| neg. 700-1899 | 2349 | 0.34 | 1269 | 0.36 |
| neg. 705-2814 | 2171 | 0.32 | 1308 | 0.37 |
| | | | | |
| pos. 1810 | 287386 | 41.90 | 5291 | 1.50 |
| pos. 1851 | 55348 | 8.07 | 1666 | 0.47 |

Im vorliegenden Beispiel wurde die Störanfälligkeit des erfindungsgemäßen Tests durch freies IgG untersucht. Die linke Ergebnisspalte zeigt, dass der Test durch freies IgG, das in einer Humanserumprobe immer vorhanden ist, nicht gestört wird, so dass eine hohe Testsensitivität erreicht wird. Der Capture-Antikörper, der gegen humanes, aggregiertes IgG gerichtet ist und eine niedrige Affinität aufweist, bindet freies IgG nicht oder nur in zu vernachlässigender Weise. An der Festphase wird somit nur aggregiertes IgG gebunden, das dann wiederum mittels Bindung an ein multimeres/ multivalentes Rubella-Antigen ("Rubella-like-Partikel") und Detektion durch einen ruthenylierten Anti-Rubella-MAK als anti-Rubella-spezifisches IgG nachgewiesen wird.

In der rechten Spalte in Beispiel 2 sind dagegen die Ergebnisse für eine Testführung gemäß dem Stand der Technik dargestellt. Im Stand der Technik diskriminiert der Capture-Antikörper nicht zwischen aggregiertem und nicht-aggregiertem, d.h. freiem IgG. Es wird folglich sowohl freies als auch aggregiertes IgG an die Festphase gebunden. Dies führt bei der anschließenden Bindung des Rubella-Antigens und der Nachweisreaktion offensichtlich dazu, dass die Sensitivität des Tests leidet. Die Probe MC4 weist einen Wert von 24,79 für die erfindungsgemäße Testführung auf, während dieselbe Probe mit der Methode des Standes der Technik nur auf einen Wert von 1,71 kommt, d.h. der Cutoff-Index von 1 nur knapp überschritten wird.

Sehr deutlich wird der Unterschied in den letzten beiden Zeilen der Ergebnistabelle, insbesondere in der letzten Zeile. Das Serum ,,pos. 1851" ist mit der Methode der Erfindung deutlich anti-Rubella-positiv (Wert: 8,07), während die Methode des Standes der Technik dieselbe Probe als negativ, d.h. falsch negativ klassifiziert (Wert 0,47, also unter dem Cut-off-Index von 1).

Die mangelnde Sensitivität des Verfahren des Standes der Technik könnte somit fatale Folgen haben, da positive Proben falsch negativ nachgewiesen werden.

Die Ergebnisse zeigen klar, dass die Verwendung eines erfindungsgemäßen Rezeptorantikörpers eine empfindlichere Detektion von anti-Rubella-Antikörpern verglichen mit einem Rezeptor-Antikörper des Standes der Technik ermöglicht.

### Beispiel 3: Detektion von Anti-Rubella-IgG-Antikörpern in menschlichen Humanserumproben, welche Rheumafaktor enthalten.

Käufliche Humanserumproben (Bioclinical Partners), welche Rheumafaktor enthalten, wurden auf anti-Rubella-IgG-Antikörper mit dem in Beispiel 1 beschriebenen erfindungsgemäßen Verfahren untersucht.

Ein Zweischritt-Immunassay (Cobas Core, Rubella IgG recomb II), des Standes der Technik wurde als Vergleichsversuch durchgeführt.

| Schwelle | Erfindungsgemäßer Einschritt-Immunassay coi<1 neg coi≥1 pos | | Zweischritt-Immunassay <10 U/ml neg ≥10 IU/ml pos | Rheumafaktor |
|---|---|---|---|---|
| | 3254 Counts | | 10 IU/ml | |
| Probe | Counts | COI | IU/ml | IU/ml |
| | | | | |
| BCP 9808-114-04673 | 141077 | 43.35 | 103.76 | 24 |
| BCP 9808-114-04674 | 765 | 0.24 | 3.53 | 353 |
| BCP 9808-114-04675 | 173111 | 53.19 | 208.43 | 429 |
| BCP 9808-114-04676 | 167319 | 51.41 | 82.80 | 186 |
| BCP 9808-114-04677 | 153119 | 47.05 | 124.57 | 324 |
| BCP 9808-114-04678 | 82728 | 25.42 | 42.28 | 189 |
| BCP 9808-114-04679 | 164793 | 50.64 | >300 | 400 |
| BCP 9808-114-04680 | 171594 | 52.73 | 204.08 | 185 |
| BCP 9808-114-04681 | 5696 | 1.75 | 11.18 | 56 |
| BCP 9808-114-04682 | 64006 | 19.67 | 65.89 | 272 |
| BCP 9808-114-04683 | 167373 | 51.43 | 146.24 | 56 |
| BCP 9808-114-04684 | 138037 | 42.42 | 71.94 | 40 |
| BCP 9808-114-04685 | 166660 | 51.21 | 187.39 | 139 |
| BCP 9808-114-04686 | 79085 | 24.30 | 85.52 | 315 |

Die Ergebnisse zeigen klar, dass das erfindungsgemäße Verfahren die Proben trotz der Anwesenheit des Rheumafaktors korrekt klassifiziert.

## Patentansprüche

1. Verfahren zur Bestimmung eines Analyten in einer Probe, umfassend die Schritte:
(a) Inkontaktbringen der Probe mit einem ersten analytspezifischen Rezeptor und einem zweiten analytspezifischen Rezeptor, wobei einer der beiden Rezeptoren an eine Festphase gebunden ist oder an eine Festphase bindefähig ist und der andere Rezeptor eine signalgebende Gruppe trägt oder mit einer signalgebenden Gruppe bindefähig ist, und
(b) Nachweisen des Vorhandenseins oder/und der Menge des Analyten durch Bestimmung der signalgebenden Gruppe auf der Festphase,
**dadurch gekennzeichnet, dass**
der erste analytspezifische Rezeptor mindestens zwei Bindestellen für den Analyten enthält und der zweite analytspezifische Rezeptor an eine Anordnung aus mindestens zwei Analytmolekülen, welche an den ersten Rezeptor gebunden sind, bindet und diese Anordnung von einem einzelnen Analytmolekül unterscheiden kann und der Analyt ein antigenspezifischer Antikörper ist.

2. Verfahren nach Anspruch 1, **dadurch gekennzeichnet, dass** der Analyt ein antigenspezifischer Antikörper der Immunglobulinklasse G ist.

3. Verfahren nach nach Anspruch 1 oder 2, **dadurch gekennzeichnet, dass** der erste Rezeptor mindestens zwei verschiedene Bindungsstellen, mindestens zwei gleiche Bindungsstellen oder eine Kombination von verschiedenen und gleichen Bindungsstellen für den Analyten enthält, bevorzugt, dass der erste Rezeptor ein oligomerer oder ein multimerer Rezeptor ist.

4. Verfahren nach einem der vorhergehenden Ansprüche, **dadurch gekennzeichnet, dass** der erste Rezeptor ein Antigen umfasst, welches mindestens zwei Epitope als Bindestellen für den Analyten enthält.

5. Verfahren nach einem der vorhergehenden Ansprüche, wobei der erste Rezeptor in Form von Rezeptorvorläufern bereitgestellt wird.

6. Verfahren nach Anspruch 5, wobei die Rezeptorvorläufer derartig beschaffen sind, dass sie während oder vor dem Schritt (a) miteinander assoziieren oder aneinander binden können, um den ersten Rezeptor zu bilden.

7. Verfahren nach einem der vorhergehenden Ansprüche, **dadurch gekennzeichnet, dass** der zweite Rezeptor ein Rheumafaktor-ähnlicher Antikörper ist.

8. Verfahren nach einem der vorhergehenden Ansprüche, **dadurch gekennzeichnet, dass** der zweite Rezeptor ein Antikörper mit wenigstens zwei Paratopen ist.

9. Verfahren nach einem der vorhergehenden Ansprüche, wobei der zweite Rezeptor ein Antikörper mit niedriger Affinität und hoher Avidität zum Analyten ist.

10. Verfahren nach ein der vorhergehenden Ansprüche, wobei Schritt (a) in einem Reaktionsansatz ohne Waschschritt durchgeführt wird.

11. Verfahren nach einem der vorhergehenden Ansprüche, welches ein diagnostisches Verfahren ist.

12. Kit, umfassend einen ersten analytspezifischen Rezeptor und einen zweiten analytspezifischen Rezeptor, wobei einer der beiden Rezeptoren an eine Festphase gebunden ist oder an eine Festphase bindefähig ist und der andere Rezeptor eine signalgebende Gruppe trägt oder mit einer signalgebenden Gruppe bindefähig ist,
**dadurch gekennzeichnet, dass**
der erste analytspezifische Rezeptor mindestens zwei Bindestellen für den Analyten enthält und der zweite analytspezifische Rezeptor an eine Anordnung aus mindestens zwei Analytmolekülen, welche an den ersten Rezeptor gebunden sind, bindet und diese Anordnung von einem einzelnen Analytmolekül unterscheiden kann und der Analyt ein antigenspezifischer Antikörper ist.

13. Kit nach Anspruch 12, wobei der erste analytspezifische Rezeptor und der zweite analytspezifische Rezeptor zur Anwendung in einem Reaktionsansatz formuliert werden.

14. Verwendung eines analytspezifischen Rezeptors, welcher mindestens zwei Bindestellen für den Analyten enthält, in einem Verfahren zur Bestimmung eines Analyten in einer Probe nach einem der Ansprüche 1 bis 11.

15. Verwendung gemäß Anspruch 14 in einem diagnostischen Verfahren.

## Claims

1. A method for determining an analyte in a sample, comprising the steps:
(a) contacting the sample with a first analyte-specific receptor and a second analyte-specific receptor, one of the two receptors being bound to a solid phase or being capable of binding to a solid phase and the other receptor bearing a signal-generating group or being capable of binding with a signal-generating group, and
(b) detecting the presence and/or quantity of the analyte by determining the signal-generating group on the solid phase,
**characterised in that**
the first analyte-specific receptor contains at least two binding sites for the analyte and the second analyte-specific receptor binds to an arrangement of at least two analyte molecules which are bound to the first receptor and this arrangement may differ from an individual analyte molecule and the analyte is an antigen-specific antibody.

2. A method according to claim 1, **characterised in that** the analyte is an antigen-specific antibody of immunoglobulin class G.

3. A method according to claim 1 or claim 2, **characterised in that** the first receptor contains at least two different binding sites, at least two identical binding sites or a combination of identical and different binding sites for the analyte, preferably **in that** the first receptor is an oligomeric or multimeric receptor.

4. A method according to any one of the preceding claims, **characterised in that** the first receptor comprises an antigen which contains at least two epitopes as binding sites for the analyte.

5. A method according to any one of the preceding claims, wherein the first receptor is provided in the form of receptor precursors.

6. A method according to claim 5, wherein the receptor precursors are of a nature such that, during or before step (a), they are capable of associating with or binding to one another in order to form the first receptor.

7. A method according to any one of the preceding claims, **characterised in that** the second receptor is a rheumatoid factor-like antibody.

8. A method according to any one of the preceding claims, **characterised in that** the second receptor is an antibody with at least two paratopes.

9. A method according to any one of the preceding claims, wherein the second receptor is an antibody with low affinity and elevated avidity for the analyte.

10. A method according to any one of the preceding claims, wherein step (a) is carried out in a reaction mixture without a washing step.

11. A method according to any one of the preceding claims which is a diagnostic method.

12. A kit comprising a first analyte-specific receptor and a second analyte-specific receptor, one of the two receptors being bound to a solid phase or being capable of binding to a solid phase and the other receptor bearing a signal-generating group or being capable of binding with a signal-generating group, **characterised in that**
the first analyte-specific receptor contains at least two binding sites for the analyte and the second analyte-specific receptor binds to an arrangement of at least two analyte molecules which are bound to the first receptor and this arrangement may differ from an individual analyte molecule and the analyte is an antigen-specific antibody.

13. A kit according to claim 12, wherein the first analyte-specific receptor and the second analyte-specific receptor are formulated for use in a reaction mixture.

14. Use of an analyte-specific receptor which contains at least two binding sites for the analyte in a method for determining an analyte in a sample according to any one of claims 1 to 11.

15. Use according to claim 14 in a diagnostic method.

## Revendications

1. Procédé pour déterminer un analyte dans un échantillon, comprenant les étapes :
(a) mise en contact de l'échantillon avec un premier récepteur spécifique d'analyte et un deuxième récepteur spécifique d'analyte, où l'un des deux récepteurs est lié à une phase solide ou peut être lié à une phase solide et l'autre récepteur porte un groupe émettant un signal ou peut être lié à un groupe émettant un signal, et
(b) mise en évidence de la présence et/ou de la quantité de l'analyte par
détermination du groupe émettant un signal sur la phase solide,
**caractérisé en ce que**
le premier récepteur spécifique d'analyte contient au moins deux sites de liaison pour l'analyte et le deuxième récepteur spécifique d'analyte se lie à un agencement d'au moins deux molécules d'analyte qui sont liées au premier récepteur et peut distinguer cet agencement d'une seule molécule d'analyte et l'analyte est un anticorps spécifique d'antigène.

2. Procédé selon la revendication 1 **caractérisé en ce que** l'analyte est un anticorps spécifique d'antigène de la classe des immunoglobulines G.

3. Procédé selon la revendication 1 ou 2 **caractérisé en ce que** le premier récepteur contient au moins deux sites de liaison différents, au moins deux sites de liaison identiques ou une combinaison de sites de liaison différents et identiques pour l'analyte, de préférence **en ce que** le premier récepteur est un récepteur oligomère ou un récepteur multimère.

4. Procédé selon l'une des revendications précédentes **caractérisé en ce que** le premier récepteur comprend un antigène qui contient au moins deux épitopes comme sites de liaison pour l'analyte.

5. Procédé selon l'une des revendications précédentes où le premier récepteur est préparé sous forme de précurseurs de récepteur.

6. Procédé selon la revendication 5 où les précurseurs de récepteur sont tels qu'ils peuvent s'associer entre eux ou se lier entre eux pendant ou avant l'étape (a) pour former le premier récepteur.

7. Procédé selon l'une des revendications précédentes **caractérisé en ce que** le deuxième récepteur est un anticorps analogue à un facteur rhumatoïde.

8. Procédé selon l'une des revendications précédentes **caractérisé en ce que** le deuxième récepteur est un anticorps ayant au moins deux paratopes.

9. Procédé selon l'une des revendications précédentes où le deuxième récepteur est un anticorps à faible affmité et grande avidité pour l'analyte.

10. Procédé selon l'une des revendications précédentes où l'étape (a) est conduite dans une masse réactionnelle sans étape de lavage.

11. Procédé selon l'une des revendications précédentes qui est un procédé diagnostique.

12. Kit comprenant un premier récepteur spécifique d'analyte et un deuxième récepteur spécifique d'analyte, où l'un des deux récepteurs est lié à une phase solide ou peut être lié à une phase solide et l'autre récepteur porte un groupe émettant un signal ou peut être lié à un groupe émettant un signal, **caractérisé en ce que**
le premier récepteur spécifique d'analyte contient au moins deux sites de liaison pour l'analyte et le deuxième récepteur spécifique d'analyte se lie à un agencement d'au moins deux molécules d'analyte qui sont liées au premier récepteur et peut distinguer cet agencement d'une seule molécule d'analyte et l'analyte est un anticorps spécifique d'antigène.

13. Kit selon la revendication 12 où le premier récepteur spécifique d'analyte et le deuxième récepteur spécifique d'analyte sont formulés pour une utilisation dans une masse réactionnelle.

14. Utilisation d'un récepteur spécifique d'analyte qui contient au moins deux sites de liaison pour l'analyte dans un procédé pour déterminer un analyte dans un échantillon selon l'une des revendications 1 à 11.

15. Utilisation selon la revendication 14 dans un procédé diagnostique.
